# EUROPÄISCHE PATENTSCHRIFT

(11) **EP 2 844 197 B1**
(45) Veröffentlichungstag und Bekanntmachung des Hinweises auf die Patenterteilung: **07.10.2020**
(21) Anmeldenummer: 13719127.6
(22) Anmeldetag: 30.04.2013
(51) Int. Cl.: A61F 5/01, A61F 5/02

(54) **SPANNVORRICHTUNG FÜR ORTHESEN**
FASTENING DEVICE FOR ORTHOSES
DISPOSITIF TENDEUR POUR ORTHÈSES

(30) Priorität: 02.05.2012 DE 102012009214
(43) Veröffentlichungstag der Anmeldung: 11.03.2015
(73) Patentinhaber: Bauerfeind AG, 07937 Zeulenroda-Triebes (DE)
(72) Erfinder: ROEBELT, Gerhard, 07950 Zeulenroda-Triebes (DE); BAETZ, Ronny, 07937 Vogtländisches Oberland OT Pöllwitz (DE); STIER, Gerald, 07957 Langenwetzendorf (DE)
(74) Vertreter: Wohlfahrt, Jan Günther
(86) Internationale Anmeldenummer: PCT/EP2013/059021
(87) Internationale Veröffentlichungsnummer: WO 2013/164353

(56) Entgegenhaltungen:
- WO-A1-99/65428
- DE-A1-102010 035 309
- US-A1- 2009 082 707
- US-A1- 2010 168 630
- US-A1- 2011 295 169

## Beschreibung

Die Erfindung betrifft eine neuartige Spannvorrichtung für Orthesen zur Stützung und Funktionserhaltung des menschlichen Körpers, besonders körperumgreifende Rückenorthesen. Die Spannvorrichtung erlaubt ein individuelles, segmentweises Anpassen der Stützwirkung durch individuell separat ausgebildete Flaschenzüge, die an einer als Zentralelement ausgestalteten Rückenplatte individuell und höhenverstellbar angebracht werden können.

Orthesen sind therapeutische Hilfsmittel zur Stabilisierung oder Unterstützung der Bewegungsfunktion von Körperteilen, beispielsweise von Becken und Wirbelsäule. Der Einsatz der Orthesen kann direkt posttraumatisch oder postoperativ oder konservativ erfolgen. Bei der Verwendung werden die Orthesen in der Regel um das Körperteil, beispielsweise um die Hüfte herum, angelegt und so gürtelartig geschlossen, dass ein stabilisierender Druck auf die zu stabilisierende Körperregion ausgeübt wird. Beispielsweise bei Lumbalorthesen kann es erforderlich sein, eine bestimmte Wirbelsäulenkrümmung (Lordose) zu fixieren, um weitere Schäden an der Wirbelsäule zu verhindern oder einen postoperativen Zustand zu stabilisieren, um die Heilung zu verbessern.

Bekannte Orthesen, beispielsweise Lumbalorthesengürtel, weisen häufig Spannvorrichtungen auf, mit deren Hilfe die Spannung/der Druck der umgelegten Orthese auf das Körperteil kontrolliert erhöht werden kann. Dazu kann eine Flaschenzugvorrichtung vorgesehen sein. Diese erstreckt sich bekanntermaßen im Wesentlichen über die gesamte Breite der Orthese mit dem Ziel, die Zugkraft über die gesamte Breite des Orthesengürtels zu vergleichmäßigen. Bekannte, auf Flaschenzügen basierende Spannvorrichtungen weisen dazu ein loses Zugseil auf, das zu einer Seite der Orthese hin verläuft. Es hat sich herausgestellt, dass derart aufgebaute Orthesen bei der Verwendung mit Nachteilen behaftet sind: Durch die durch den bekannten Flaschenzugmechanismus bedingte Vergleichmäßigung der Zugkraft ist eine individuelle Anpassung der Spannung und damit der Stützwirkung auf ein individuelles Behandlungsziel nicht möglich. Gleichzeitig kommt es durch das Spannen bekannter Flaschenzugvorrichtungen über einseitige Zugseile zu einer einseitigen Kraftwirkung an der Orthese und damit zu deren Verziehen oder Verrutschen.

Eine verbesserte Spannvorrichtung mit Flaschenzügen ist aus der DE 10 2010 035 309 A1 bekannt.

Die Erfindung hat sich die Aufgabe gestellt, Spannvorrichtungen für Orthesen weiterzuentwickeln und zu verbessern, um die bekannten Nachteile zu vermeiden. Das der Erfindung zugrunde liegende technische Problem liegt in der Bereitstellung einer verbesserten Spannvorrichtung für eine Orthese, die insbesondere in ihrer Stützfunktion besser an die Anforderungen der Therapie oder Prophylaxe angepasst werden kann. Insbesondere sollen durch die Orthese die wirkenden Kräfte zielgerichtet und individuell auf bestimmte Körperpunkte, insbesondere Wirbelsäulenabschnitte, geleitet werden.

Das technische Problem wird gelöst durch eine Spannvorrichtung nach Anspruch 1.

Das technische Problem wird insbesondere gelöst durch eine Spannvorrichtung für eine Orthese, aufweisend mindestens ein erstes Seitenelement an einem ersten Ende und mindestens ein zweites Seitenelement an einem gegenüberliegenden zweiten Ende und mindestens ein dazwischen angeordnetes Zentralelement, wobei erstes und zweites Seitenelement über das Zentralelement durch mindestens zwei zwischen Zentralelement und erstem Seitenelement verlaufenden ersten Flaschenzügen und mindestens zwei zwischen Zentralelement und zweitem Seitenelement verlaufenden zweiten Flaschenzügen miteinander verbunden sind, wobei die Ansatzstellen der Flaschenzüge an das Zentralelement über lösbare mechanische Koppelmittel höhenverstellbar sind.

Die Erfindung betrifft also eine Spannvorrichtung, wobei das erste Seitenelement über mindestens zwei Flaschenzüge mit dem Zentralelement verbunden ist und das zweite Seitenelement über mindestens zwei weitere Flaschenzüge mit dem Zentralelement verbunden ist, wobei die Ansatzpunkte der mindestens vier Flaschenzüge an das Zentralelement über lösbare mechanische Koppelmittel unabhängig voneinander höhenverstellbar sind.

Es hat sich gezeigt, dass sich die Kraftvektoren einer Orthese, insbesondere Rückenorthese individuell und zielgerichtet auf den Ort von Rückenbeschwerden ausgerichtet werden können, wenn Flaschenzüge eingesetzt werden, die individuell an einer Rückenplatte, die als Zentralelement dient, höhenverstellbar angebracht werden können, so dass die Flaschenzüge dort die größte Spannwirkung an der Wirbelsäule entfalten können, wo sie an der Rückenplatte angebracht werden. So ist es mit einer erfindungsgemäßen Spannvorrichtung insbesondere möglich bei einer Versorgung eines Patienten mit einer Rückenorthese gezielt und punktgenau bestimmte Wirbelsäulenabschnitte zu belasten oder zu entlasten und somit ein Maximum an therapeutischer Wirksamkeit zu erzielen.

Erfindungsgemäß bevorzugt erfolgt das Anbringen der Flaschenzüge an das Zentralelement über Koppelmittel. Erfindungsgemäß bevorzugt umfasst ein Koppelmittel ein erstes Koppelelement und ein zweites Koppelelement, wobei sich das erste Koppelelement an einem Flaschenzug befindet und das zweite Koppelelement sich an dem Zentralelement befindet.

Erfindungsgemäß bevorzugt handelt es sich bei dem ersten oder zweiten Koppelelement um ein Einhängelement. Erfindungsgemäß bevorzugt handelt es sich bei dem zweiten oder ersten Koppelelement um eine Aussparung.

Erfindungsgemäß bevorzugt erfolgt das Anbringen der Flaschenzüge an das Zentralelement durch Einhängen von Einhängelementen der Flaschenzüge in Aussparungen des Zentralelements oder durch Einhängen von Einhängelementen des Zentralelements in Aussparungen der Flaschenzüge.

Die Einhängeelemente können beispielsweise Hintergriffelemente oder Rastelemente sein. Die Aussparungen können beispielsweise Löcher sein. Die Löcher können jegliche geeignete Form haben, beispielsweise rund oder oval. Dem Fachmann sind geeignete Formen bekannt.

In einer bevorzugten Ausführungsform sind die lösbaren mechanischen Koppelmittel als Hintergriffelemente oder Rastelemente ausgestaltet.

In einer bevorzugten Ausführungsform sind die Koppelelemente des Zentralelements links und rechts des die Wirbelsäule abdeckenden Bereichs angeordnet.

Erfindungsgemäß sind die Ansatzpunkte der mindestens zwei ersten Flaschenzüge und der mindestens zwei zweiten Flaschenzüge unabhängig voneinander höhenverstellbar. In einer bevorzugten Ausführungsform sind die Ansatzpunkte aller Flaschenzüge der Spannvorrichtung unabhängig voneinander höhenverstellbar.

Das erste Seitenelement ist über mindestens zwei Flaschenzüge mit dem Zentralelement verbunden. Das zweite Seitenelement ist über mindestens zwei Flaschenzüge mit dem Zentralelement verbunden. Erfindungsgemäß ist also das erste Seitenelement über mindestens zwei Flaschenzüge mit dem Zentralelement verbunden und das zweite Seitenelement über mindestens zwei Flaschenzüge mit dem Zentralelement verbunden, wobei die Ansatzpunkte mindestens der vier Flaschenzüge an das Zentralelement über lösbare mechanische Koppelmittel unabhängig voneinander höhenverstellbar sind.

In einer bevorzugten Ausführungsform ist die Spannwirkung der Spannvorrichtung durch Verringerung des Abstands der Seitenelemente zueinander über die Flaschenzüge erzielbar.

In einer bevorzugten Ausführungsform ist jeder Flaschenzug einzeln spannbar und/oder lösbar. Bevorzugt sind also die einzelnen Flaschenzüge nicht nur unabhängig voneinander höhenverstellbar sondern auch unabhängig voneinander spannbar und/oder lösbar.

In einer bevorzugten Ausführungsform ist also das erste Seitenelement über mindestens zwei Flaschenzüge mit dem Zentralelement verbunden und das zweite Seitenelement über mindestens zwei Flaschenzüge mit dem Zentralelement verbunden, wobei die Ansatzpunkte mindestens der vier Flaschenzüge an das Zentralelement über lösbare mechanische Koppelmittel unabhängig voneinander höhenverstellbar sind und wobei die mindestens vier Flaschenzüge unabhängig voneinander spannbar und/oder lösbar sind.

In einer bevorzugten Ausführungsform sind die Flaschenzüge als einfacher oder mehrfacher Flaschenzug mit mindestens einem Zugseilumlenkung und einem Zugseilverankerung ausgebildet. In einer bevorzugten Ausführungsform sind die Flaschenzüge als einfacher Flaschenzug ausgebildet. In einer bevorzugten Ausführungsform sind die Flaschenzüge als mehrfacher Flaschenzug ausgebildet.

In einer bevorzugten Ausführungsform verlaufen die Zugseile des Flaschenzugs in einem zwischen Seitenelement und Zentralelement verlaufenden Zugseiltunnel aus plastischem flexiblem Werkstoff. Bevorzugt ist der Zuseiltunnel elastisch. Dies hat den Vorteil, dass der Zugseiltunnel bei Spannung der Zugseile kaum oder gar nicht störende Falten wirft.

In einer bevorzugten Ausführungsform weist der Zugseiltunnel in seinem Verlauf zwischen Seitenelement und Zentralelement einen zusätzlichen Tunnelzweig auf, worin das lose Ende des Zugseils geführt wird.

In einer bevorzugten Ausführungsform sind die Seitenelemente klettbar, beispielsweise an ein Orthesengestrick.

In einer bevorzugten Ausführungsform ist das Zentralelement als Rückenplatte so gestaltet, dass es sich an die Kontur der Wirbelsäulenanatomie anformen lässt. In einer alternativen Ausführungsform ist das Zentralelement als Überbrückungsplatte ausgestaltet. Das Zentralelement kann aber auch so ausgestaltet sein, so dass es sich als Rückenplatte und/oder Überbrückungsplatte verwenden lässt.

Ist das Zentralelement als Überbrückungsplatte ausgestaltet sind bevorzugt entsprechende Kanäle vorgesehen, in denen starre Stäbe eingeschoben werden können.

In einer bevorzugten Ausführungsform ist das Zentralelement so gestaltet, dass es entlang der Wirbelsäule hohl gelegt ist. Dadurch werden Druckstellen an der Wirbelsäule vermieden.

Das technische Problem wird auch gelöst durch eine Spannvorrichtung für eine Orthese, die vor allem dadurch gekennzeichnet ist, dass sie mindestens ein Spannsegment aufweist, insbesondere mindestens zwei, besonders bevorzugt mehrere getrennte, separate, besonders im Wesentlichen parallel verlaufende, zueinander benachbarte, besonders unmittelbar angrenzende im Wesentlichen gleichartige Spannsegmente aufweist, wobei diese Spannsegmente voneinander unabhängig über separate Flaschenzüge spannbar sind und die Flaschenzüge an einer als Zentralelement ausgestalten Rückenplatte individuell und höhenverstellbar angebracht werden können.

Eine Ausführungsform ist also eine erfindungsgemäße Spannvorrichtung, enthaltend mindestens ein Spannsegment, insbesondere mindestens zwei Spannsegmente, bevorzugt mehrere separate, unabhängig spannbare Spannsegmente, die jeweils ein erstes Seitenelement an einem ersten Ende und ein zweites Seitenelement an einem gegenüberliegenden zweiten Ende und ein dazwischen angeordnetes Zentralelement aufweisen, wobei erstes und zweites Seitenelement eines Spannsegments über das Zentralelement dieses Spannsegments durch mindestens einen zwischen Zentralelement und erstem Seitenelement verlaufenden ersten Flaschenzug und mindestens einen zwischen Zentralelement und zweitem Seitenelement verlaufenden zweiten Flaschenzug miteinander verbunden sind.

Eine Ausführungsform ist also eine erfindungsgemäße Spannvorrichtung, enthaltend mehrere separate, unabhängig spannbare Spannsegmente, die jeweils ein erstes Seitenelement an einem ersten Ende und ein zweites Seitenelement an einem gegenüberliegenden zweiten Ende und ein dazwischen angeordnetes Zentralelement aufweisen, wobei erstes und zweites Seitenelement eines Spannsegments über das Zentralelement dieses Spannsegments durch einen zwischen Zentralelement und erstem Seitenelement verlaufenden ersten Flaschenzug und einen zwischen Zentralelement und zweitem Seitenelement verlaufenden zweiten Flaschenzug höhenverstellbar miteinander verbunden sind.

Bevorzugt sind das erste und das zweite Seitenelement eines Spannsegments über das Zentralelement dieses Spannsegments durch einen zwischen Zentralelement und erstem Seitenelement verlaufenden ersten Flaschenzug und einen zwischen Zentralelement und zweitem Seitenelement verlaufenden zweiten Flaschenzug miteinander verbunden.

Die Seitenelemente, das Zentralelement und die Flaschenzüge eines Spannsegments sind bevorzugt wie weiter oben beschrieben ausgeführt.

Die Spannsegmente weisen jeweils ein erstes Seitenelement an einem ersten Ende der Spannvorrichtung und ein zweites Seitenelement an einem gegenüberliegenden zweiten Ende der Spannvorrichtung auf. Dazwischen ist ein bevorzugt mittig zwischen dem ersten und zweiten Seitenelement ein Zentralelement angeordnet. Die Seitenelemente sind über das Zentralelement miteinander mechanisch verbunden, und zwar über jeweils mindestens einen, bevorzugt einen zwischen dem Zentralelement und dem ersten Seitenelement verlaufenden ersten Flaschenzug und jeweils mindestens einen, bevorzugt einen zwischen dem Zentralelement und dem zweiten Seitenelement verlaufenden zweiten Flaschenzug, und bilden so ein Spannsegment.

Bevorzugt sind die Zentralelemente der Spannsegmente miteinander verbunden.

Bevorzugt sind die Zentralelemente der mindestens zwei, insbesondere mehreren Spannsegmente über Verbindungselemente miteinander verbunden. Alternativ können die Zentralelemente der Spannsegmente auch zusammen ein langes einstückiges Zentralelement bilden. Die Zentralelemente der mindestens zwei, insbesondere mehreren Spannsegmente können beispielsweise über Stäbe, Schienen oder Röhren, beispielsweise aus Metall, insbesondere Aluminium oder Kunststoff miteinander verbunden sein. Es sind natürlich aber auch Schraubverbindungen, Druckknopfverbindungen oder Hakenverbindungen zwischen jeweils zwei Zentralelementen möglich. Die Zentralelemente von mindestens zwei Spannsegementen können starr oder flexibel, beispielsweise über ein Gelenk miteinander verbunden sein.

Es versteht sich, dass die Zentralelemente der mindestens zwei, insbesondere mehreren Spannsegmente übereinander miteinander verbunden sind, also das Zentralelement des obersten und des untersten Spannsegments mit jeweils einem weiteren Zentralelement verbunden ist und bei mindestens drei Spannsegmenten, das mindestens eine dazwischen liegende Zentralelement mit zwei weiteren Zentralelementen verbunden ist.

Die Zentralelemente der mindestens zwei, insbesondere mehreren Spannsegmente können aber auch nicht direkt miteinander verbunden sein, sondern nur über die Flaschenzüge und/oder die Rückenorthese.

Im angelegten Zustand ist die Spannvorrichtung zusammen mit der Orthese um das Körperteil herum gelegt und die beiden Enden der Spannvorrichtung stehen miteinander in kraftschlüssiger Verbindung; die Spannvorrichtung ist wie ein Gürtel um das Körperteil geschlossen. Die Erfindung sieht innerhalb eines einzelnen Spannsegments zu beiden Seiten eines Zentralelements jeweils mindestens einen Flaschenzug vor, der sich zu den Seitenelementen hin erstreckt. Die sich gegenüber liegenden Flaschenzüge eines Spannsegments können bei der Benutzung der angelegten Orthese über ein beidseitiges Ziehen der losen Enden ihrer beiden Zugseile in entgegengesetzter Richtung zirkulär gespannt werden. Beim Spannen wird der Abstand zwischen den Seitenelementen und damit den beiden Enden der Spannvorrichtung zueinander zu verkürzt und eine das Körperteil umlaufende zirkuläre Spannung erzeugt. Dadurch wird vorteilhafterweise eine zum Zentralelement symmetrische Krafteinwirkung erreicht. Die zirkuläre Spannung kann so unmittelbar symmetrisch gleichmäßig auf beiden Seiten des Zentralelements wirken, sodass dieses beim Spannen nicht aus seiner Position verschoben wird. Ein Verwinden oder ein Verschieben der Spannvorrichtung oder der mit der Spannvorrichtung verbundenen Orthese beim Spannen wird wirksam verhindert.

In einer bevorzugten Ausführungsform weist das Zentralelement eines Spannsegments mindestens zwei übereinander liegende Koppelmittel für den ersten Flaschenzug zum Verbinden des ersten Seitenelements und mindestens zwei übereinander liegende Koppelmittel für den zweiten Flaschenzug zum Verbinden des ersten Seitenelements auf. Besonders bevorzugt weist das Zentralelement eines Spannsegments mindestens drei übereinander liegende Koppelmittel für den ersten Flaschenzug zum Verbinden des ersten Seitenelements und mindestens drei übereinander liegende Koppelmittel für den zweien Flaschenzug zum Verbinden des ersten Seitenelements auf.

Bevorzugt weist das Zentralelement eines Spannsegments drei, vier, fünf, sechs oder sieben übereinander liegende Koppelmittel für den ersten Flaschenzug zum Verbinden des ersten Seitenelements und drei, vier, fünf, sechs oder sieben übereinander liegende Koppelmittel für den zweien Flaschenzug zum Verbinden des ersten Seitenelements auf.

In einer erfindungsgemäßen Ausgestaltung sind Flaschenzüge mit mehrfacher Umlenkung ausgebildet. Die Flaschenzüge sind jeweils an dem Zentralelement und an dem ersten beziehungsweise zweiten Seitenelement ein und desselben Spannsegmentes verankert. Der Flaschenzug eines Spannsegments hat keine Verankerung oder Umlenkpunkte an einem anderen insbesondere benachbarten Spannsegment. Erfindungsgemäß weist also jedes Spannsegment eigene, zu den anderen Spannsegmenten getrennte Flaschenzüge auf.

In Abkehr vom Stand der Technik sieht die Erfindung also auch vor, mehrere Flaschenzüge vorzugsweise im Wesentlichen parallel zueinander unabhängig in separaten Spannsegmenten anzuordnen. Jeder Flaschenzug greift jeweils ausschließlich an den ihm zugeordneten Zentralelement und Seitenelementen eines Spannsegments an, wobei der Angriffspunkt an das Zentralelement höhenverstellbar ist. Jedem einzelnen Spannsegment ist also in dieser Ausgestaltung bevorzugt ein einziges individuell einstellbares Paar von Flaschenzügen zugeordnet. Jeder Flaschenzug ermöglicht in Verbindung mit dem ihm gegenüberliegenden, zum anderen Ende hin weisenden Flaschenzug eine segmentweise individuell einstellbare symmetrische zirkuläre Spannwirkung, wobei diese Spannwirkung durch die Höhenverstellbarkeit in Bezug auf das Zentralelement individuell und zielgerichtet an einen bestimmten Punkt des Zentralelements hingeleitet werden kann.

Die Erfindung sieht bevorzugt vor, dass zur Realisierung des Zugseilantriebs in Form eines Flaschenzugs an dem Zentralelement zu beiden Seiten hin pro Segment jeweils, bevorzugt genau ein Zugseilumlenkelement zum Umlenken des Zugseils des jeweiligen Flaschenzugs oder wahlweise oder zusätzlich, bevorzugt genau eine Verankerung des Zugseilendes vorgesehen ist. Damit vermeidet die Erfindung die Vergleichmäßigung der Kraftwirkung eines Flaschenzugs, über größere Abschnitte beziehungsweise die gesamte Breite des Orthesengürtels bei bekannten, mehreren nebeneinander liegenden Zugseilumlenkelementen oder Verankerungen des Zugseils. Vielmehr besitzt der erfindungsgemäße Flaschenzug bevorzugt jeweils genau einen Verankerungspunkt mit dem Zentralelement und dem Seitenelement des Spannsegments; der Verankerungspunkt ist jeweils als Zugseilumlenkelement, bevorzugt als Umlenkrolle, oder als Verankerungspunkt, worin das Zugseil ortsfest fixiert ist, ausgebildet. Erfindungsgemäß bevorzugt wird also pro Spannsegment jeweils in einem einzigen Kraftansatzpunkt über den Flaschenzug Kraft ausgeübt. Der Kraftansatzpunkt ist, je nach Auslegung des Flaschenzugs, als Zugseilumlenkung oder, alternativ oder zusätzlich, als Zugseilverankerung ausgebildet. Es ist in einer besonderen Ausführung der Erfindung vorgesehen, dass an Zentralelement und/oder Seitenelement mindestens zwei beabstandete optionale Verankerungspunkte vorbereitet sind, die zur individuellen Anpassung der Spannvorrichtung jeweils als alternative Kraftansatzpunkte gewählt werden können.

In besonderer Ausgestaltung weist die Spannvorrichtung zumindest zwei, bevorzugt drei oder vier oder fünf, separat spannbare Spannsegmente auf. Bevorzugte Varianten weisen genau drei, vier oder fünf aneinander angrenzende Spannsegmente auf, denen jeweils ein Flaschenzugpaar zugeordnet ist. Diese Varianten sind also an drei, vier oder fünf Segmenten separat spannbar.

Die segmentweise Krafteinleitung in einem einzigen Kraftansatzpunkt innerhalb eines Segments erlaubt, vor allem im Zusammenhang mit der Verwendung der Spannvorrichtung an oder in einer Rückenorthese eine wirbelsegmentsgenaue Krafteinleitung. Vorzugsweise sind in dieser Ausgestaltung der Erfindung einem Spannsegment zumindest ein Wirbel oder eine Wirbelgruppe zugeordnet. Bevorzugt bewirkt ein einzelnes Spannsegment eine überwiegende Krafteinwirkung an oder im Bereich genau eines Wirbels oder Wirbelabschnitts und ein anderes Spannsegment bewirkt eine überwiegende Krafteinwirkung an oder im Bereich genau eines anderen Wirbels oder Wirbelabschnitts.

In einer besonderen Ausgestaltung ist zusätzlich vorgesehen, dass zur erfindungsgemäßen segmentweisen Krafteinwirkung mindestens ein Spannsegment zusätzlich, bevorzugt an oder im Bereich seines Zentralelements, mindestens eine zum Körperteil gewandte Pelotte aus bevorzugt elastischem Polstermaterial aufweist. Diese Pelotte ist bevorzugt derart ausgestaltet, dass sie die Krafteinwirkung dieses Spannsegments gezielt auf eine bestimmte Körperregion richtet. In einer bevorzugten Variante die Pelotte an der Spannvorrichtung individuell ab- oder aufrüstbar. Die Pelotte ist bevorzugt in ihrer Form und/oder ihrem Werkstoff zur Modifikation der Wirkungsweise der Vermittlung der an dem Spannsegment austausch und/oder einstellbar. In einer besonderen Variante kann einem Spannsegment eine Pelotte zugeordnet sein, die bestimmte Weichteilstrukturen des Körperteils, in Verbindung mit der gezielt einstellbaren Krafteinwirkung des Spannsegments, individuell massiert und stimuliert. Beispielsweise kann so ein Triggerpunkt eines Muskels gezielt stimuliert werden, um beispielsweise eine gezielte Spannung oder Entspannung des Muskels zu erreichen. Solche gezielt segmentweise einstellbaren und auslösbaren Einwirkungen auf das Körperteil sind durch bekannte spannbare Orthesen nicht erreichbar.

Bevorzugt ist die Pelotte verstellbar anbringbar, insbesondere höhenverstellbar, so dass die Pelotte auf dem Zentralelement am gewählten Angriffspunkt mindestens eines Flaschenzuges positioniert ist.

In besonderer Ausgestaltung ist zusätzlich vorgesehen, dass zumindest zwei benachbarte Spannsegmente der Spannvorrichtung miteinander mechanisch verbunden sind. Hierbei ist bevorzugt eine starre Kopplung vorgesehen. Alternativ bevorzugt ist eine flexible Kupplung, besonders in Form eines flexiblen, beispielsweise elastischen, Bandes. In besonderer Ausgestaltung davon sind zumindest die Seitenelemente zweier unmittelbar benachbarter Segmente mechanisch miteinander verbunden, um mindestens eine integrale Seitenbrücke aus mindestens zwei Seitenelementen zu bilden. In einer besonderen Ausgestaltung sind die Seitenelemente aller vorhandenen Segmente miteinander in dieser Weise zu einer einzigen Seitenbrücke verbunden. Die Seitenbrücken sind in einer besonderen Variante einstückig ausgebildet.

In besonderer Ausgestaltung davon sind, alternativ oder zusätzlich, zumindest die Zentralelemente zweier unmittelbar benachbarter Segmente mechanisch miteinander verbunden, um mindestens eine integrale Zentralbrücke aus mindestens zwei Zentralelementen zu bilden. In einer besonderen Ausgestaltung sind die Zentralelemente aller vorhandenen Segmente miteinander in dieser Weise zu einer einzigen Zentralbrücke verbunden. Die Zentralbrücke kann einstückig ausgebildet sein.

In besonderer Ausgestaltung weist die integrale Zentralbrücke zusätzlich mindestens ein senkrecht zur Kraftrichtung der Flaschenzüge verlaufendes Stützelement für ein Körperteil auf oder es besteht daraus. Dieses Stützelement ist an oder in dem oder insbesondere als Zentralelement ausgebildet. Das Stützelement ist aus einem vergleichsweise unelastischen zähen und spröden Werkstoff ausgeführt. Das Stützelement kann in Form eines einfachen Stabes, beispielsweise als individuell formbarer Metallstab oder thermoplastisch formbarer Kunststoffstab ausgebildet sein. Durch die erfindungsgemäß segmentweise ausübbare Spannkraft kann dieses Stützelement individuell an das Körperteil angelegt werden, um die Stützfunktion beziehungsweise die therapeutische formgebende Funktion zu ermöglichen. Besonders ist dieses Stützelement in anatomisch und therapeutisch zweckmäßiger Weise geformt und dient unmittelbar der Stützung eines Körperteils, besonders der Wirbelsäule, das heißt im Beispiel einer Rückenorthese verläuft das Stützelement entlang der Wirbelsäule und dient damit zu deren Stützung.

Alternativ sind anstelle eines einzelnen Stützstabes zwei zueinander beabstandete stabförmige Stützelemente, die über Querbrücken, die zwischen Aussparungen des Zentralelements gebildet sind, miteinander verbunden sind, vorgesehen. Besonders im Falle einer Rückenorthese kann so die Wirbelsäule unmittelbar links und rechts der Wirbelkämme gestützt werden; eine unmittelbare Druckbelastung der Wirbelkämme wird vermieden.

In besonderer Ausgestaltung sieht die Erfindung vor, dass die Zugseile des Flaschenzugs eines ersten Spannsegments jeweils zusammen, aber getrennt und beabstandet von den Zugseilen des Flaschenzugs eines benachbarten zweiten Segments geführt werden. In einer besonderen Ausgestaltung ist dazu ein Gitterrahmen vorgesehen, der jeweils zwischen den Zentralelementen und Seitenelementen angeordnet ist, und die Zugseile eines Flaschenzugs zusammen und jeweils getrennt von den Zugseilen eines anderen Flaschenzugs beabstandet hält. Die Zugseile sind dazu entsprechend durch die Maschen des Gitterrahmens geführt. Dabei ist bevorzugt vorgesehen, dass, besonders im Falle einer Rückenorthese, der Gitterrahmen gleichzeitig als stabilisierende Beckenfassung ausgebildet ist, der gleichzeitig rotationsstabilisierend wirkt.

In besonderer Ausgestaltung der Erfindung werden die Zugseile eines Flaschenzugs in einem Zugseiltunnel geführt. Der Zugseiltunnel erstreckt sich bevorzugt zwischen dem Seitenelement und dem Zentralelement. Der Zugseiltunnel ist bevorzugt aus einem plastischen flexiblen Werkstoff ausgeführt; besonders bevorzugt ist ein elastisches Gestrick. Die Erfindung ermöglicht so eine kompakte und anwenderfreundliche Realisierung der segmentweisen individuellen Flaschenzüge. Ein Inkontaktbringen der Zugseile benachbarter Flaschenzüge und ein Verhaken mit Kleidungsstücken oder anderen Orthesenbestandteilen wird so wirkungsvoll verhindert.

In einer besonderen Variante dieser Ausgestaltung weist der Zugseiltunnel zusätzlich einen Tunnelzweig auf, der im Verlauf zwischen Seitenelement und Zentralelement abzweigt. Darin kann jeweils das lose Ende des Zugseils eines Flaschenzugs geführt werden. Durch den ebenso wie der Zugseiltunnel aus plastischem oder flexiblem Werkstoff ausbildbaren Tunnelzweig kann das lose Ende so geführt werden, dass die Gefahr des Verdrillens oder Verknotens mit den Zugseilenden benachbarter Flaschenzüge verhindert und die Anwendung der Spannvorrichtung beim Anlegen und Spannen vereinfacht wird. In einer alternativen oder zusätzlichen Ausgestaltung werden die Zugseile der Flaschenzüge jeweils getrennt in einem Abstandsgewirk geführt.

In einer besonderen Ausgestaltung sind die Zugseile unmittelbar in das Gestrick einer Orthese eingelegt. Dazu weist das Gestrick der Orthese vorzugsweise tunnelartige Aussparungen oder Laschen auf, die eine getrennte Führung der Zugseile ermöglichen. Zusätzliche Maßnahmen wie der in der vorbezeichneten Ausgestaltung vorgesehene Gitterrahmen zur Beabstandung der Zugseile benachbarter Flaschenzüge erübrigen sich in dieser Ausgestaltung. In der vereinfachten Ausgestaltung einer bereits in die Gestrickorthese integrierten erfindungsgemäßen Spannvorrichtung ist eine hierin beschriebene Auf- und Abrüstbarkeit der Spannvorrichtung auf eine stehende, insbesondere herkömmliche Orthese, besonders Gestrickorthese, nicht erforderlich; die erfindungsgemäße Spannvorrichtung ist vielmehr ein integraler Bestandteil einer entsprechenden neuartigen hierin beschriebenen erfindungsgemäßen Orthese.

In einer besonderen Ausgestaltung mündet das lose Ende eines Zugseils in einen Griff, worin es fixiert ist. Über den Griff kann der Benutzer das lose Ende des Zugseils eines Flaschenzugs spannen. Es ist vorgesehen, dass der Griff im Bereich des mit dem Flaschenzug verbundenen Seitenelements, das heißt im Bereich der jeweiligen Enden der Spannvorrichtung, lösbar fixierbar ist, um die Spannung aufrecht zu erhalten. Zum Voreinstellen der Zuglänge des Zugseilendes kann das Griffstück eine Klemmvorrichtung aufweisen. Hiermit kann der "Arbeitspunkt" jedes Flaschenzugs segmentweise eingestellt werden, um die individuelle Anpassung der Orthese sicherzustellen. Die zeitweise Fixierung des Griffs an der Spannvorrichtung erfolgt in an sich bekannter Weise über Verklettung oder Verknüpfung.

Alternativ kann anstelle eines ortsveränderlich fixierbaren Griffs im Bereich der Seitenelemente ein, vorzugsweise rastbaren, Aufrollmechanismus für die jeweiligen Zugseile vorgesehen sein.

Seitenelement und/oder Zentralelement weisen Verbindungsmittel auf, um mit der Orthese, gegebenenfalls lösbar, verbunden zu werden. Die Erfindung erlaubt so die Auf- beziehungsweise Abrüstung der Spannvorrichtung auch auf eine bestehende Orthese. Besonders ist die bestehende Orthese eine herkömmliche Gestrickorthese, besonders eine Staborthese, die aufgrund ihrer Eigenelastizität um das Köperteil herumgeführt und in an sich bekannter Weise über Laschen geschlossen wird. Im Falle einer Rückenorthese kann die erfindungsgemäße Spannvorrichtung im Bereich der Rückenseite und der Hüftseite auf die Orthese aufgerüstet werden.

Die erfindungsgemäße Spannvorrichtung erlaubt eine individuelle und punktgenaue Anpassung der Spannkraft einer Orthese über einen weiten Bereich. Da die stabilisierende beziehungsweise fixierende Wirkung der Orthese im Wesentlichen vollständig durch die Spannvorrichtung selbst übernommen wird, braucht eine darunterliegende elastische in an sich bekannter Weise ausgebildete Gestrickorthese selbst keine vollständige Spann- oder Stützwirkung mehr ausüben. Es kann daher vorteilhafterweise ein Gestrick verwendet werden, das über einen großen Umfangsbereich, im Falle einer Rückenorthese beispielsweise sowohl bei schmaler Taille als auch bei großem Bauch- beziehungsweise Brustumfang, eingesetzt werden kann, ohne dass jeweils individuell an die Körpergröße angepasste Gestrickorthesen bereitgestellt werden müssen. Die Anpassung an den Körperumfang kann dabei jeweils bevorzugt ausschließlich über die Einstellung der Flaschenzüge, besonders also über die segmentweise Einstellung der "Arbeitspunkte" der losen Enden der Flaschenzüge, vorgenommen werden.

Die vorliegende Erfindung offenbart auch eine Rückenplatte für eine Rückenorthese, wobei die Rückenplatte links und rechts einer gedachten senkrechten Mittellinie mindestens zwei, bevorzugt mindestens drei übereinander liegende Kopplungselemente aufweist, wobei die Kopplungselemente zum wieder lösbaren Koppeln eines Flaschenzugs geeignet sind.

Bei der Rückenplatte kann es sich insbesondere um ein Zentralelement einer erfindungsgemäßen Spannvorrichtung, eines erfindungsgemäßen Spannsegments, mehrerer erfindungsgemäßer Spannsegmente oder einer erfindungsgemäßen Orthese handeln. Wie für ein Zentralelement bereits beschrieben, können durch eine offenbarte Rückenplatte Spannkräfte indivduell und punktgenau Angelegt werden, indem zum Beispiel mindestens ein erster Flaschenzug mit einem gewünschten linken Kopplungselement verbunden wird und mindestens ein zweier Flaschenzug mit einem gewünschten rechten Kopplungselement verbunden wird.

In einer bevorzugten Ausführungsform weist die Rückenplatte mindestens zwei Rückenplattensegmente auf, die über Verbindungselemente miteinander verbunden sind. Die Rückenplattensegmente, können beispielsweise über Stäbe, Schienen oder Röhren, beispielsweise aus Metall, insbesondere Aluminium oder Kunststoff miteinander verbunden sein. Es sind natürlich aber auch Schraubverbindungen, Druckknopfverbindungen oder Hakenverbindungen zwischen jeweils zwei Zentralelementen möglich. Die Rückenplattensegmente können starr oder flexibel, beispielsweise über ein Gelenk miteinander verbunden sein.

In einer bevorzugten Ausführungsform handelt es sich bei den Kopplungselementen um Löcher zum Einrasten oder Hintergreifen von einem Kopplungsgegenelement eines Flaschenzugs.

Die vorliegende Erfindung betrifft auch eine Orthese, enthaltend eine erfindungsgemäße Spannvorrichtung.

Die vorliegende Erfindung betrifft auch die Verwendung einer erfindungsgemäßen Spannvorrichtung zur segmentweisen und positionsgenauen Steuerung der Spann- oder Stützfunktion einer Orthese.

Weitere Ausgestaltungsformen kann der Fachmann ohne weiteres von der DE 10 2010 035 309 A1 ableiten.

Die Erfindung wird durch die nachfolgenden Figuren und Figurenbeschreibungen näher illustriert, ohne dass diese beschränkend zu verstehen wären.
- Figur 1: zeigt eine Ausführungsform einer erfindungsgemäßen Spannvorrichtung.
- Figur 2: einen Ausschnitt einer Ausführungsform einer erfindungsgemäßen Spannvorrichtung.
- Figuren 3a bis 3c: zeigen verschiedene Ausführungsformen einer Rückenplatte oder eines erfindungsgemäßen Zentralelements.

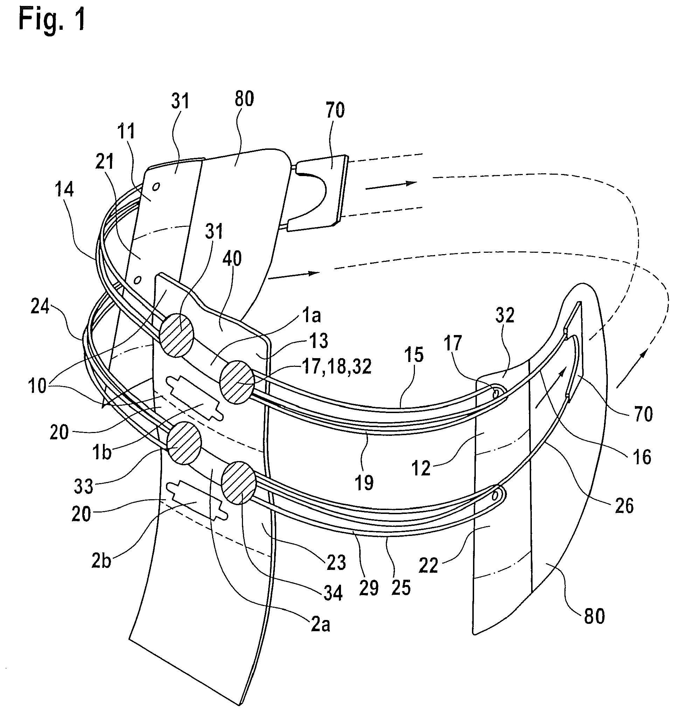

Figur 1 zeigt eine Ausgestaltung der erfindungsgemäßen Spannvorrichtung, angepasst zur Verwendung an einer Rückenorthese zur Stabilisierung der Wirbelsäule. Die Ansicht zeigt aus Gründen der Übersichtlichkeit nicht vollständig alle wiederkehrenden Strukturen.

Pro Spannsegment (10) sind, rechts und links des Zentralelements (13), jeweils zwei Zugseilantriebe (14, 15) in Form von Flaschenzügen verbunden, die jeweils mit dem, in der Figur linksseitig und im Hintergrund dargestellten, Seitenelement (11) und dem, rechtsseitig und im Vordergrund dargestellten, zweiten Seitenelement (12) mechanisch verbunden sind. Die Flaschenzüge (14, 15) weisen jeweils Zugseile (19), Zugseilumlenkungen (17) und Zugseilverankerungen (18) auf, um jeweils einen mehrfachen Flaschenzug zu bilden. Pro Spannsegment sind die Flaschenzüge (14, 15) so in jeweils genau einem Kraftansatzpunkt (17, 18) an Zentralelement (13) und Seitenelement (11, 12) verankert. In der Figur sind die zu Seitenbrücken (31, 32) verbundenen Seitenelemente und zu einer Zentralbrücke (40) verbundenen Zentralelemente über Trennlinien voneinander unterscheidbar gemacht. Die Flaschenzüge (14, 15) des ersten Spannsegments (10) weisen jeweils lose Seilenden (16) auf, über die die Flaschenzüge gespannt werden können, wodurch sich der Abstand zwischen dem Zentralelement (13) und den Seitenelementen (11, 12) jeweils verkürzt, um zu spannen.

Entsprechend weisen die benachbarten Spannsegmente (20) Flaschenzüge (24, 25) auf mit Zugseilen (29) und losen Enden (26). Die Flaschenzüge (24, 25) verbinden jeweils die ersten und zweiten Seitenelemente (21, 22) über das Zentralelement (23) miteinander.

Die vorliegende erfindungsgemäße Ausführungsform zeichnet sich dadurch aus, dass die Ansatzstellen, also Kraftansatzpunkte der Flaschenzüge (14, 15, 24, 25) an die Zentralelemente (13, 23) der Spannsegmente (10, 20) über lösbare mechanische Kopplungsmittel höhenverstellbar sind. Die mechanischen Kopplungsmittel umfassen in den Zentralelementen (13, 23) befindliche Langlöcher (1a, 1b, 2a, 2b) mit verkleinerten Enden und Kopplungselemente (31, 32, 33, 34), die als Zugseilumlenkung (17) und Zugseilbefestigung (18) ausgebildet sind, und die mit einem Hintergriffelement in die Langlöcher (1a, 1b, 2a, 2b) der Zentralelementen (13, 23) eingehakt werden können. Die Kopplungselemente (31, 32) des ersten Spannsegments (10) können unabhängig voneinander entweder in das obere Langloch (1a) des ersten Spannsegments (10) oder in das untere Langloch (1b) des ersten Spannsegments (10) eingehängt werden. Die Kopplungselemente (33, 34) des zweiten Spannsegments (20) können unabhängig voneinander entweder in das obere Langloch (2a) des zweiten Spannsegments (20) oder in das untere Langloch (2b) des zweiten Spannsegments (20) eingehängt werden. Somit wird in erfindungsgemäßer und vorteilhafter Weise erreicht, dass die Kraftansatzpunkte der einzelnen Flaschenzüge (14, 15, 24, 25) an die Zentralelemente (13, 23) höhenverstellbar sind und somit in einem gewünschten Bereich wirken können. Natürlich kann ein Segment auch mehr als zwei Langlöcher aufweisen, beispielsweise drei bis sechs Langlöcher, so dass die Höhenverstellbarkeit noch variabler und genauer wird.

Die untereinander liegenden separaten Spannsegmente (10, 20) sind mechanisch miteinander verkoppelt. Dabei ist jeweils das erste Seitenelement (11) des ersten Spannsegments (10) mit dem ersten Seitenelement (21) des zweiten Spannsegments (20) zu einer gemeinsamen einstückigen Seitenbrücke (31) verkoppelt. Entsprechend ist das zweite Seitenelement (21) des ersten Spannsegments mit dem zweiten Seitenelement (22) des zweiten Spannsegments (20) zu einer einstückigen Seitenbrücke (32) verkoppelt. Ebenso ist das Zentralelement (13) des ersten Spannsegments (10) mit dem Zentralelement (23) des zweiten Spannsegments zu einer einstückigen Zentralbrücke (40) verkoppelt. Trotz der mechanischen Verkopplung mechanischer Spannsegmente (10, 20) miteinander über die Seitenbrücken (31, 32) und die Zentralbrücke (40) ist über die segmentweise getrennt geführten Flaschenzüge eine segmentweise individuelle Anpassung der Spannkraft möglich.

In der Ausgestaltung als Rückenorthese bildet die Zentralbrücke (40) eine anatomisch anformbare Wirbelsäulenstütze, die im angelegten Zustand der Orthese auf oder im Bereich der Wirbelsäule platziert ist.

Die rechtsseitigen und linksseitigen losen Enden (16, 26) der Flaschenzüge einzelner Spannsegmente (10, 20) münden, zueinander beabstandet, in einen gemeinsamen Griff (70). Der Griff (70) ist im Bereich der Seitenelemente/Seitenbrücken (11, 12, 31, 32) lösbar fixierbar. Durch Verschwenken des Griffs (70) beim Fixieren kann die Spannkraft auf die beiden Spannsegmente individuell verteilt werden. Die gebildeten Seitenbrücken (31, 32) laufen in der dargestellten Ausgestaltung jeweils in Verschlusslaschen (80) aus, die beim Anlegen der Orthese am Körper in Form eines Gürtels oder einer Bandage miteinander kraftschlüssig verbunden werden, wodurch der erfindungsgemäße segmentweise zirkuläre Kraftschluss erreicht wird.

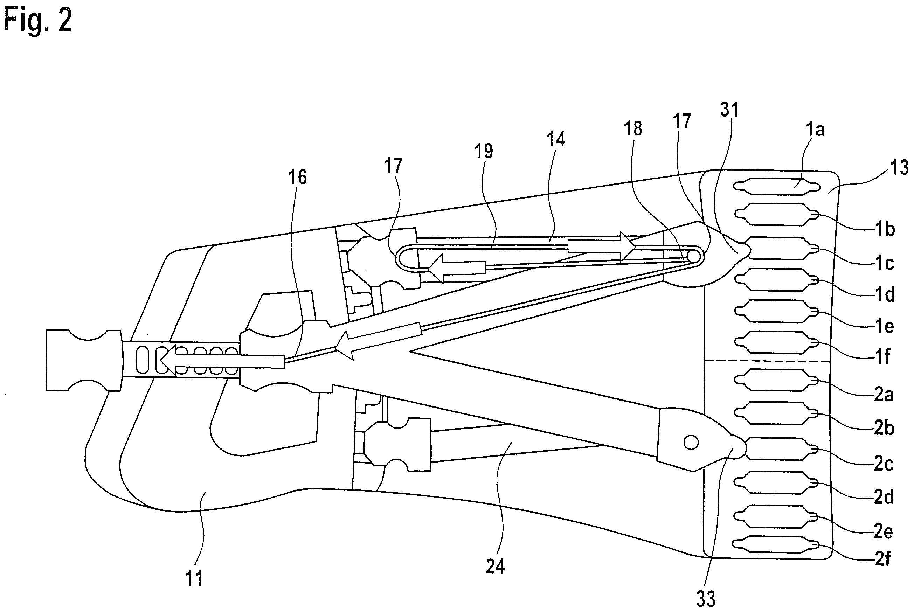

Figur 2 zeigt schematisch einen Ausschnitt aus einer weiteren Ausgestaltung der erfindungsgemäßen Spannvorrichtung. Die Ansicht zeigt aus Gründen der Übersichtlichkeit nur ein Seitenelement (11) und das Zentralelement (13). Das zweite Seitenelement würde in gleicher Weise wie das erste Seitenelement an die andere Hälfte des Zentralelements anschließen, nur gespiegelt. Das Seitenelement (11) ist mit dem Zentralelement (13) über zwei Zugseilantriebe (14, 24) in Form von Flaschenzügen mechanisch verbunden. Die Flaschenzüge (14, 24) weisen jeweils Zugseilumlenkungen (17) und Zugseilverankerungen (18) für ein Zugseil (19) auf, um jeweils einen mehrfachen Flaschenzug zu bilden. Pro Spannsegment sind die Flaschenzüge (14, 15) so in jeweils einem Kraftansatzpunkt (17, 18) an Zentralelement (13) und Seitenelement (11) verankert. Die Flaschenzüge (14, 24) weisen jeweils lose Seilenden (16) auf, über die die Flaschenzüge gespannt werden können, wodurch sich der Abstand zwischen dem Zentralelement (13) und dem Seitenelement (11) verkürzt, um zu spannen.

Die vorliegende erfindungsgemäße Ausführungsform zeichnet sich dadurch aus, dass die Ansatzstellen, also Kraftansatzpunkte der Flaschenzüge (14, 24) an das Zentralelement (13) der Spannsegmente über lösbare mechanische Kopplungsmittel höhenverstellbar sind. Die mechanischen Kopplungsmittel umfassen in den Zentralelementen (13, 23) befindliche Langlöcher (1a bis 1f, 2a bis 2fb) mit verkleinerten Enden und Kopplungselemente (31, 33), die als Zugseilumlenkung (17) und Zugseilbefestigung (18) ausgebildet sind, und die mit einem Hintergriffelement in die Langlöcher (1a bis 1f, 2a bis 2fb) des Zentralelements (13) eingehakt werden können. Das Kopplungselement (31) kann in eines der oberen Langlöcher (1a bis 1f) eingehängt werden. Das Kopplungselement (33) kann in eines der unteren Langlöcher (2a bis 2f) eingehängt werden. Somit wird in erfindungsgemäßer und vorteilhafter Weise erreicht, dass die Kraftansatzpunkte der einzelnen Flaschenzüge (14, 24) an das Zentralelement (13) höhenverstellbar sind und somit in einem gewünschten Bereich wirken können. Ein Fachmann wird ohne weiteres erkennen, das ein zweites Seitenelement auf gleiche Weise mit dem Zentralelement (13) verbunden werden kann.

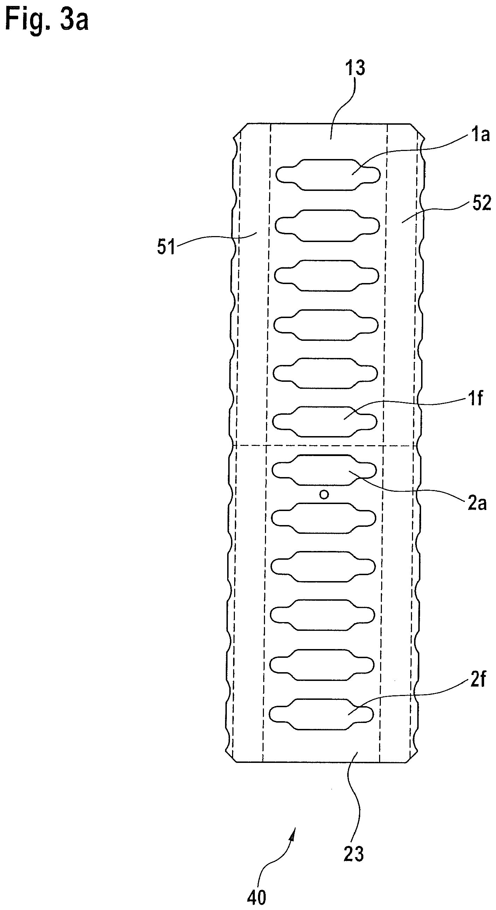

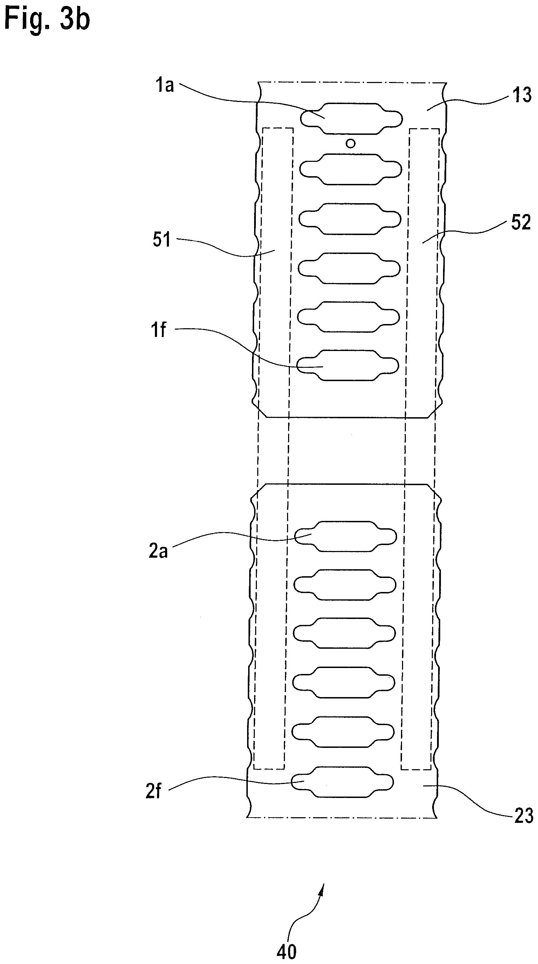

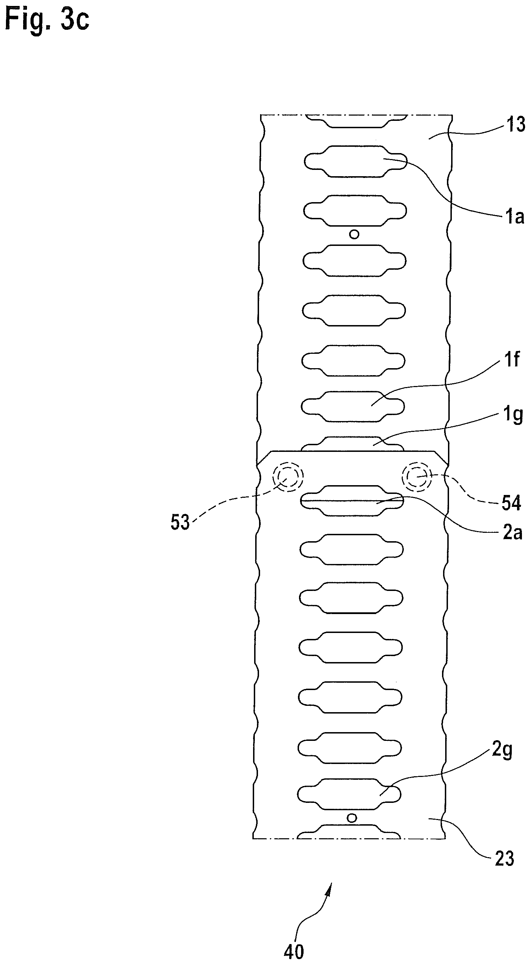

Figuren 3a bis 3c zeigen verschieden Ausführungsformen einer rückenplatte oder Zentralbrücke (40) mit zwei Zentralelementen (13, 23). Alle Zentralelemente weisen Langlöcher (1a bis 1f, 2a bis 2fb) mit verkleinerten Enden auf, in die Kopplungselemente eines Flaschenzugs eingehakt oder eingerastet werden können. In Figur 3a sind die beiden Zentralelemente (13, 23) zusammen einstückig ausgebildet. Sie sind mit zwei Aluminiumstäben oder -röhren (51, 52) verstärkt. In Figur 3 b sind die beiden Zentralelemente (13, 23) von einander getrennt und beabstandet und über die zwei Aluminiumstäben oder -röhren (51, 52) miteinander verbunden. In Figur 3c sind die beiden Zentralelemente (13, 23) von einander getrennt, überlappen sich aber teilweise und sind über zwei Verbindungselemente (53, 54), beispielsweise Clips oder Schraubenverbindungen, miteinander verbunden. Die Zentralelemente habe zusätzliche Langlöcher (1g, 2g). Die Langlöcher (1g, 2a) im Bereich der Überlappung der beiden Zentralelemente (13, 23) sind in dieser Ausführungsform nicht zum Einhängen der Kopplungselemente geeignet.

## Patentansprüche

1. Spannvorrichtung für eine Orthese, aufweisend mindestens ein erstes Seitenelement (11) an einem ersten Ende und mindestens ein zweites Seitenelement (12) an einem gegenüberliegenden zweiten Ende und mindestens ein dazwischen angeordnetes Zentralelement (13), wobei erstes und zweites Seitenelement (11,12) über das Zentralelement (13) durch mindestens zwei zwischen Zentralelement (13) und erstem Seitenelement (11) verlaufenden ersten Flaschenzügen (14, 24) und mindestens zwei zwischen Zentralelement (13) und zweitem Seitenelement (12) verlaufenden zweiten Flaschenzügen (15,25) miteinander verbunden sind, **dadurch gekennzeichnet, dass** die Ansatzpunkte der mindestens vier Flaschenzüge (14, 15, 24, 25) an das Zentralelement (13) über lösbare mechanische Koppelmittel unabhängig voneinander höhenverstellbar sind.

2. Spannvorrichtung nach Anspruch 1, enthaltend mindestens ein unabhängig spannbares Spannsegment (10), das ein erstes Seitenelement (11) an einem ersten Ende und ein zweites Seitenelement (12) an einem gegenüberliegenden zweiten Ende und ein dazwischen angeordnetes Zentralelement (13) aufweist, wobei erstes und zweites Seitenelement (11,12) des Spannsegments (10) über das Zentralelement (13) dieses Spannsegments durch einen zwischen Zentralelement (13) und erstem Seitenelement (11) verlaufenden ersten Flaschenzug (14) und einen zwischen Zentralelement (13) und zweitem Seitenelement (12) verlaufenden zweiten Flaschenzug (15) miteinander verbunden sind.

3. Spannvorrichtung nach Anspruch 1 oder Anspruch 2, enthaltend mehrere separate, unabhängig spannbare Spannsegmente (10), die jeweils ein erstes Seitenelement (11) an einem ersten Ende und ein zweites Seitenelement (12) an einem gegenüberliegenden zweiten Ende und ein dazwischen angeordnetes Zentralelement (13) aufweisen, wobei jeweils erstes und zweites Seitenelement (11,12) eines Spannsegments (10) über das Zentralelement (13) dieses Spannsegments durch einen zwischen Zentralelement (13) und erstem Seitenelement (11) verlaufenden ersten Flaschenzug (14) und einen zwischen Zentralelement (13) und zweitem Seitenelement (12) verlaufenden zweiten Flaschenzug (15) miteinander verbunden sind.

4. Spannvorrichtung nach einem der Ansprüche 2 oder 3, wobei das Zentralelement eines Spannsegments mindestens zwei übereinander liegende Koppelmittel für den ersten Flaschenzug zum Verbinden des ersten Seitenelements und mindestens zwei übereinander liegende Koppelmittel für den zweien Flaschenzug zum Verbinden des ersten Seitenelements aufweist.

5. Spannvorrichtung nach einem der Ansprüche 2 bis 4, wobei die Zentralelemente der mehreren Spannsegemente über Verbindungselemente, insbesondere Stäbe, Schraubverbindungen, Druckknopfverbindungen oder Hakenverbindungen, miteinander verbunden sind.

6. Spannvorrichtung nach einem der vorstehenden Ansprüche, wobei die lösbaren mechanischen Koppelmittel als Hintergriffelemente oder Rastelemente ausgestaltet sind.

7. Spannvorrichtung nach einem der vorstehenden Ansprüche, wobei die Spannwirkung der Spannvorrichtung durch Verringerung des Abstands der Seitenelemente (11,12) zueinander über die Flaschenzüge (14,15, 24, 25) erzielbar ist.

8. Spannvorrichtung nach einem der vorstehenden Ansprüche, wobei der Flaschenzug (14,15, 24, 25) als einfacher oder mehrfacher Flaschenzug mit mindestens einem Zugseilumlenkung (17) und einem Zugseilverankerung (18) ausgebildet ist.

9. Spannvorrichtung nach einem der vorstehenden Ansprüche, wobei die Zugseile (19) des Flaschenzugs (14,15) in einem zwischen Seitenelement (11,12) und Zentralelement (13) verlaufenden Zugseiltunnel (60) aus plastischem flexiblem Werkstoff verlaufen.

10. Spannvorrichtung nach Anspruch 9, wobei der Zugseiltunnel (60) in seinem Verlauf zwischen Seitenelement und Zentralelement einen zusätzlichen Tunnelzweig (61) aufweist, worin das lose Ende (16) des Zugseils geführt wird.

11. Orthese, enthaltend die Spannvorrichtung nach einem der vorstehenden Ansprüche.

## Claims

1. Tensioning device for an orthosis, having at least one first side element (11) on a first end, and at least one second side element (12) on an opposite second end, and at least one central element (13) arranged in between, wherein the first and second side element (11, 12) are connected to one another by means of the central element (13) by means of at least two first pulley assemblies (14, 24) extending between the central element (13) and the first side element (11) and at least two second pulley assemblies (15, 25) extending between the central element (13) and the second side element (12), **characterized in that** the leverage points of the at least four pulley assemblies (14, 15, 24, 25) on the central element (13) are height-adjustable independently from one another by means of detachable mechanical coupling means.

2. Tensioning device according to claim 1, containing at least one independently tensionable tensioning segment (10), having a first side element (11) on a first end and a second side element (12) on an opposite second end, and a central element (13) arranged in between, wherein the first and the second side element (11, 12) of the tensioning segment (10) are connected to one another by means of the central element (13) of said tensioning segment by a first pulley assembly (14) extending between the central element (13) and the first side element (11), and a second pulley assembly (15) extending between the central element (13) and the second side element (12).

3. Tensioning device according to claim 1 or claim 2, containing a plurality of separate, independently tensionable tensioning segments (10), each having a first side element (11) on a first end and a second side element (12) on an opposite second end, and a central element (13) arranged in between, wherein each of the first and second side element (11, 12) of a tensioning segment (10) are connected to one another by means of the central element (13) of said tensioning segment by a first pulley assembly (14) extending between the central element (13) and the first side element (11), and a second pulley assembly (15) extending between the central element (13) and the second side element (12).

4. Tensioning device according to one of the claims 2 or 3, wherein the central element of a tensioning segment has at least two coupling means, positioned one above the other, for the first pulley assembly for connecting the first side element, and at least two coupling means, positioned one above the other, for the second pulley assembly for connecting the first side element.

5. Tensioning device according to one of the claims 2 to 4, wherein the central elements of the plurality of tensioning segments are connected to one another by means of connection elements, particularly rods, screw connections, snap connections, or hook connections.

6. Tensioning device according to one of the previous claims, wherein the detachable mechanical coupling means are designed as latching elements or detent elements.

7. Tensioning device according to one of the previous claims, wherein the tensioning effect of the tensioning device can be achieved by decreasing the distance of the side elements (11, 12) to one another by means of the pulley assemblies (14, 15, 24, 25).

8. Tensioning device according to one of the previous claims, wherein the pulley assembly (14, 15, 24, 25) is designed as single or multiple pulley assembly with at least one pulling cable deflection (17) and one pulling cable anchoring (18).

9. Tensioning device according to one of the previous claims, wherein the pulling cables (19) of the pulley assembly (14, 15) run in a pulling cable tunnel (60), made of a plastic flexible material, which runs between side element (11, 12) and central element (13).

10. Tensioning device according to claim 9, wherein the pulling cable tunnel (60) has an additional tunnel branch (61) in its path between side element and central element, in which the loose end (16) of the pulling cable is guided.

11. Orthosis, containing the tensioning device according to one of the previous claims.

## Revendications

1. Dispositif de serrage pour une orthèse, ayant au moins un premier élément latéral (11) au niveau d'une première extrémité, et au moins un second élément latéral (12) au niveau d'une seconde extrémité opposée, et au moins un élément central (13) disposé entre ceux-ci, dans lequel le premier et second élément latéral (11, 12) sont reliés l'un avec l'autre, au moyen de l'élément central (13), par au moins deux premières poulies (14, 24) s'étendant entre l'élément central (13) et le premier élément latéral (11), et au moins deux secondes poulies (15, 25) s'étendant entre l'élément central (13) et le second élément latéral (12), **caractérisé en ce que** les points de fixation des au moins quatre poulies (14, 15, 24, 25) à l'élément central (13) sont réglables en hauteur indépendamment l'un de l'autre, par des moyens d'accouplement mécaniques détachables.

2. Dispositif de serrage selon la revendication 1, contenant au moins un segment de serrage (10) qui est indépendamment serrable et présente un premier élément latéral (11) au niveau d'une première extrémité et un second élément latéral (12) au niveau d'une seconde extrémité opposée, et un élément central (13) disposé entre ceux-ci, dans lequel le premier et second élément latéral (11, 12) du segment de serrage (10) sont reliés l'un avec l'autre, au moyen de l'élément central (13) de ce segment de serrage, par une première poulie (14) s'étendant entre l'élément central (13) et le premier élément latéral (11), et une seconde poulie (15) s'étendant entre l'élément central (13) et le second élément latéral (12).

3. Dispositif de serrage selon la revendication 1 ou la revendication 2, contenant plusieurs segments de serrage (10) séparés qui sont indépendamment serrables et respectivement présentent un premier élément latéral (11) au niveau d'une première extrémité et un second élément latéral (12) au niveau d'une seconde extrémité opposée, et un élément central (13) disposé entre ceux-ci, dans lequel en chaque cas le premier et second élément latéral (11, 12) d'un segment de serrage (10) sont reliés l'un avec l'autre, au moyen de l'élément central (13) de ce segment de serrage, par une première poulie (14) s'étendant entre l'élément central (13) et le premier élément latéral (11), et une seconde poulie (15) s'étendant entre l'élément central (13) et le second élément latéral (12).

4. Dispositif de serrage selon l'une quelconque des revendications 2 ou 3, dans lequel l'élément central d'un segment de serrage présente au moins deux moyens d'accouplement superposés pour la première poulie, afin de relier le premier élément latéral, et au moins deux moyens d'accouplement superposés pour la seconde poulie, afin de relier le premier élément latéral.

5. Dispositif de serrage selon l'une quelconque des revendications 2 à 4, dans lequel les éléments centraux des plusieurs segments de serrage sont reliés l'un avec l'autre au moyen des éléments de liaison, notamment des tiges, liaisons par vissage, liaisons par bouton-pression ou liaisons par crochet.

6. Dispositif de serrage selon l'une quelconque des revendications précédentes, dans lequel les moyens d'accouplement mécaniques détachables sont réalisés sous forme des éléments par prise en arrière ou des éléments d'encliquetage.

7. Dispositif de serrage selon l'une quelconque des revendications précédentes, dans lequel l'action de serrage du dispositif de serrage peut être effectuée en réduisant la distance mutuelle des éléments latéraux (11, 12) au moyen des poulies (14, 15, 24, 25).

8. Dispositif de serrage selon l'une quelconque des revendications précédentes, dans lequel la poulie (14, 15, 24, 25) est configurée en tant que poulie simple ou multiple avec au moins un renvoi de câble de traction (17) et un ancrage de câble de traction (18).

9. Dispositif de serrage selon l'une quelconque des revendications précédentes, dans lequel les câbles de traction (19) de la poulie (14, 15) passent dans un conduit de câble de traction (60) en matière plastique flexible, le conduit s'étendant entre l'élément latéral (11, 12) et l'élément central (13).

10. Dispositif de serrage selon la revendication 9, dans lequel le conduit de câble de traction (60), dans son passage entre l'élément latéral et l'élément central, comprend une branche de conduit (61) additionnelle, dans laquelle l'extrémité (16) libre du câble de traction est guidée.

11. Orthèse comprenant le dispositif de serrage selon l'une quelconque des revendications précédentes.
